# FASCICULE DE BREVET EUROPEEN

(11) **EP 3 080 664 B1**
(45) Date de publication et mention de la délivrance du brevet: **29.08.2018**
(21) Numéro de dépôt: 14824899.0
(22) Date de dépôt: 10.12.2014
(51) Int. Cl.: G03B 15/00, G02B 13/06, G03B 17/17, G01N 21/01, G01N 21/84, G03B 37/00, G01N 21/85, G01N 21/952

(54) **DISPOSITIF ET PROCEDE D'IMAGERIE D'UN OBJET**
DEVICE AND METHOD FOR IMAGING AN OBJECT
VORRICHTUNG UND VERFAHREN ZUR ABBILDUNG EINES OBJEKTS

(30) Priorité: 10.12.2013 FR 1302875; 09.12.2014 FR 1462125
(43) Date de publication de la demande: 19.10.2016
(73) Titulaire: Shakti, 13013 Marseille (FR); Arvalis Institut du végétal, 75116 Paris (FR); Biogemma, 75001 Paris (FR)
(72) Inventeur: CHAZALLET, Frédéric, F-13013 Marseille (FR)
(74) Mandataire: Marchand, André
(86) Numéro de dépôt international: PCT/FR2014/053250
(87) Numéro de publication internationale: WO 2015/086988

(56) Documents cités:
- WO-A1-2006/087176
- WO-A1-2009/067622
- US-A- 6 128 143
- US-A1- 2002 146 248
- US-A1- 2009 079 838
- US-B1- 6 333 826
- US-B1- 6 449 103
- JIA J: "Seed maize quality inspection with machine vision", SPIE - COMPUTER VISION FOR INDUSTRY,, vol. 1989, 24 juin 1993 (1993-06-24), pages 288-295, XP002507235, ISBN: 978-0-8194-1238-6
- GENOVESE ET AL: "360-deg Measurement on Tubular Samples with Axial Stereogrammetry", SEM ANNUAL CONFERENCE AND EXPOSITION ON EXPERIMENTAL AND APPLIED MECHANICS 2006 : JUNE 4 - 7, 2006, SAINT LOUIS, MISSOURI, USA, SOCIETY FOR EXPERIMENTAL MECHANICS, US, vol. 3, 1 January 2006 (2006-01-01), pages 1172-1181, XP009504368, ISBN: 978-0-912053-95-0

## Description

La présente invention est relative à un dispositif de prise d'images d'un objet, en particulier un dispositif de prise d'images périphériques d'un objet de forme allongée, et à un procédé de prise d'images d'un objet à l'aide d'un tel dispositif.

### ETAT DE LA TECHNIQUE

Il est connu de déterminer une propriété d'un objet par traitement d'images de l'objet.

La demande de brevet US2009046890 décrit un procédé et un système d'analyse d'images numériques d'un épi de maïs, qui permettent de déterminer au moins une propriété de l'épi et des grains de maïs, notamment le nombre et la dimension des grains.

Le système comporte un capteur d'images tel qu'une caméra CCD, qui délivre des images à des moyens de traitement d'images qui appliquent aux images divers algorithmes de traitement tel qu'un filtrage ou une recherche de contours, par exemple.

Cependant, la prise d'images d'épis posés sur un support tel qu'un convoyeur, ne permet pas de disposer d'une vue complète de l'épi et ne permet qu'une estimation approximative des propriétés de l'épi et de ses grains.

De tels procédés et systèmes de prise d'images d'un objet ne sont pas adaptés pour déterminer, in situ, des propriétés d'un objet dans un environnement confiné, à partir d'images de l'objet, en particulier pour déterminer, sur son lieu de croissance (sur pied), une propriété d'une partie d'un végétal telle qu'un épi de maïs.

En effet, un objet disposé dans un environnement confiné ne peut souvent pas faire l'objet de prise d'images de toute sa surface périphérique, en raison de la distance minimale d'écartement entre l'objet et l'appareil de prise d'images qui est nécessaire pour obtenir des images de l'objet, et/ou en raison d'obstacles entourant l'objet et empêchant de faire tourner l'appareil de prise d'images autour de l'objet.

Exemples de dispositifs conventionnels de prise d'images d'un objet sont divulgués par US 6449103 B1.

### EXPOSÉ DE L'INVENTION

Un objectif de l'invention est de proposer un procédé et un dispositif de prise d'images d'un objet, en particulier un procédé et un dispositif de prise d'images périphériques d'un objet de forme allongée, qui soient améliorés et/ou qui remédient, en partie au moins, aux lacunes ou inconvénients des procédés et dispositifs connus de prise d'images d'un objet.

Selon un aspect de l'invention, il est proposé un procédé de prise d'images d'un objet selon la rev. indépendante 16. Selon un autre aspect de l'invention, il est proposé un dispositif d'acquisition d'images d'un objet selon la rev. indépendante 1. La réflexion d'images d'observation « radiale » de tout ou partie de la surface périphérique de l'objet, parallèlement à l'axe de l'optique réflective et/ou à l'axe longitudinal de l'objet, par l'optique réflective évidée au travers de laquelle peut s'étendre et se déplacer l'objet, facilite la prise d'images « panoramiques » de toute la périphérie d'au moins une portion longitudinale - ou « tranche » - de l'objet, in situ, notamment lorsque l'objet est entouré d'obstacles proches.

Lorsque les dimensions respectives de l'objet, notamment l'allongement de l'objet, et de l'optique réflective ne permettent pas d'obtenir, en une seule fois, une image périphérique complète de l'objet, la prise d'image(s) peut être répétée le nombre de fois nécessaire pour « balayer » toute la périphérie de l'objet, en déplaçant l'objet au travers de l'évidemment de l'optique réflective - par un mouvement relatif du dispositif par rapport à l'objet qui peut rester fixe - entre deux prises d'image(s) successives, puis en « raccordant » deux à deux les images successivement prises.

Le déplacement du dispositif par rapport à l'objet est de préférence effectué en maintenant l'objet sensiblement centré dans l'évidemment, et/ou en maintenant l'axe de l'optique réflective sensiblement confondu avec l'axe longitudinal de l'objet - ou du moins de la partie d'objet à imager.

A cet effet, le dispositif peut comporter des moyens de centrage de l'objet dans l'évidemment, en particulier des organes d'appui sur l'objet qui sont montés mobiles par rapport à l'optique réflective selon des axes « radiaux » s'étendant dans un plan perpendiculaire à l'axe de l'optique réflective.

Pour obtenir un dispositif particulièrement compact, l'optique réflective s'inscrivant (s'étendant) à l'intérieur d'un premier cylindre et l'évidemment s'inscrivant (s'étendant) à l'intérieur d'un second cylindre coaxial au premier cylindre - et de plus petit rayon -, on peut dimensionner l'optique réflective de telle sorte que le rayon du premier cylindre soit inférieur ou égal au triple ou au double du rayon du second cylindre.

Par exemple, du rayon du second cylindre peut être de l'ordre de 2 à 5 centimètres, et la valeur du rayon du premier cylindre peut être égale à celle du rayon du second cylindre augmentée de 1 à 3 centimètres environ.

Le dispositif comporte de préférence une fenêtre transparente de forme cylindrique, en particulier de section circulaire, autour de laquelle s'étend l'optique réflective, ce qui permet notamment d'éviter le contact entre l'objet et l'optique réflective, ainsi que le masquage de zones de l'image par une partie de l'objet masquant (interrompant) le faisceau réfléchi par l'optique réflective.

L'optique réflective peut comporter une unique surface réfléchissante qui est gauche, c'est-à-dire non plane, et qui présente une symétrie de révolution selon un axe de révolution (qui constitue alors ledit axe de symétrie).

Dans ce cas, on dispose de préférence la surface réfléchissante de façon telle que l'axe de révolution de la surface réfléchissante soit voisin de (et parallèle à) - en particulier sensiblement confondu avec - l'axe longitudinal de la partie de l'objet qui est entourée par la surface réfléchissante.

Alternativement, l'optique réflective peut comporter plusieurs surfaces réfléchissantes qui peuvent être gauches (en particulier légèrement convexes) ou planes, et qui sont de préférence régulièrement espacées/disposées autour d'un axe (qui est désigné dans la présente par l'expression « axe de symétrie ») qui peut être confondu avec l'axe longitudinal de la partie de l'objet qui est entourée par les surfaces réfléchissantes.

Dans ce cas, le nombre de surfaces réfléchissantes est généralement au moins égal à trois, en particulier au moins égal à quatre, par exemple égal à huit.

Dans le cas notamment de surfaces réfléchissantes planes, les surfaces réfléchissantes peuvent être de dimensions identiques et de formes identiques, en particulier de forme sensiblement trapézoïdale.

Les surfaces réfléchissantes planes sont inclinées par rapport à l'axe de symétrie des surfaces réfléchissantes, et/ou par rapport par rapport à l'axe longitudinal de la partie de l'objet qui est entourée par la (les) surface(s) réfléchissante(s), avec un angle aigu d'inclinaison qui est commun aux surfaces réfléchissantes. De la même façon, la (ou les) surface(s) réfléchissante(s) gauche(s) est (sont) inclinée(s) par rapport à l'axe de symétrie ou de révolution, avec un angle aigu d'inclinaison, de sorte que la (ou les) surface(s) réfléchissante(s) est (sont) dirigée(s) vers l'axe de symétrie ou de révolution, et vers l'axe longitudinal de la partie de l'objet qui est entourée par la (les) surface(s) réfléchissante(s).

Cet angle d'inclinaison peut être sensiblement égal à 45 degrés. Lorsque cet angle d'inclinaison est inférieur à 45 degrés, cela conduit à un grandissement de l'image de l'objet qui est inférieur à 1, ce qui peut permettre d'améliorer la compacité du système au détriment éventuel de sa résolution.

La (ou les) surface(s) réfléchissante(s) gauche(s) s'étend(ent) de préférence le long d'une surface tronconique.

La (ou les) surface(s) réfléchissante(s) peu(ven)t être partiellement réfléchissante(s) et partiellement transmissive(s) ou transparente(s), afin de permettre le passage d'un flux lumineux d'éclairage de la surface de l'objet, au travers de la (ou des) surface(s) réfléchissante(s).

Pour capter des images périphériques de l'objet réfléchies par la (ou les) surface(s) réfléchissante(s), on peut disposer un imageur unique comportant un objectif dont le diamètre est de préférence sensiblement égal à la plus grande dimension extérieure de l'optique réflective constituée de la (ou des) surface(s) réfléchissante(s), et dont l'axe optique est voisin de (et sensiblement parallèle à) - en particulier sensiblement confondu avec - l'axe longitudinal de la partie de l'objet qui est entourée par l'optique réflective, et sensiblement confondu avec l'axe de symétrie ou de révolution.

Pour utiliser un imageur unique de plus petites dimensions, on peut disposer un dispositif de repliement de faisceau sur le trajet optique entre l'imageur et la (ou les) surface(s) réfléchissante(s).

Le dispositif de repliement peut comporter une seconde optique réflective évidée s'étendant autour d'un axe confondu avec l'axe de la première optique réflective évidée, ainsi qu'une troisième optique réflective coaxiale avec les première et seconde optiques réflectives, de sorte que les trois optiques réflectives réfléchissent des images périphériques de l'objet sensiblement parallèlement à l'axe de la première optique réflective, et à plus faible distance de cet axe.

Avec un imageur unique, l'image obtenue de la périphérie d'une portion longitudinale - ou tranche - de l'objet, s'inscrit dans une couronne, notamment lorsque la surface réfléchissante est tronconique et qu'une partie de l'objet s'étend le long de l'axe de symétrie de l'optique réflective.

L'image obtenue peut être séparée en deux images pour une optique gauche (non plane par morceaux) lorsqu'aucune partie de l'objet ne coupe (ne passe par) l'axe de symétrie de l'optique réflective.

Il est alors préférable d'utiliser une optique plane (ou légèrement convexe) par morceaux pour assembler sur l'imageur unique des images segmentées obtenus par une ou des séries de réflexions sur des plans, donc sans ou avec très peu d'aberrations et/ou distorsions.

Une telle image peut faire l'objet d'un traitement mathématique la transformant en une image en forme de bande correspondant à une vue de la périphérie de la tranche « mise à plat », et d'autres traitements permettant de déterminer des propriétés de l'objet.

Alternativement, on peut disposer plusieurs imageurs dont les axes optiques respectifs sont distants de (et parallèles à) l'axe de symétrie ou de révolution de la première optique réflective.

Dans ce cas, chacune des images obtenues correspond à une partie seulement de la périphérie de l'objet, et peut être en forme de secteur de couronne; ces images peuvent présenter (deux à deux) un recouvrement (partiel) et peuvent alors faire l'objet d'un traitement permettant de « raccorder » ces images deux à deux et d'obtenir une image de la périphérie complète d'au moins une « tranche » de l'objet.

Ces images en forme de secteur peuvent être transformées en des images rectangulaires en éliminant des images les portions d'image proches des « jointures » séparant deux faces réfléchissantes adjacentes, qui sont projetées sur deux faces réfléchissantes adjacentes.

Selon un mode de réalisation, on peut disposer les axes optiques respectifs des imageurs de façon régulière autour de l'axe de symétrie ou de révolution de la première optique réflective.

Dans ce cas notamment, les imageurs peuvent être montés sur au moins un support en forme de couronne présentant un (second) évidemment centré sur l'axe de symétrie/révolution de la première optique réflective.

Un tel support évidé d'imageurs peut contribuer à délimiter, avec la première optique réflective évidée, et le cas échéant avec la fenêtre transparente de forme cylindrique ou tubulaire, une cavité apte à recevoir (la totalité de) l'objet à observer/imager, en particulier une cavité de forme cylindrique allongée selon l'axe de symétrie ou de révolution de la première optique réflective, et dont le diamètre est supérieur à celui du plus petit cylindre dans lequel peut s'inscrire l'objet.

Chaque imageur peut comporter un capteur d'images matriciel, en particulier un capteur de technologie CCD ou CMOS, qui peut comporter un ou plusieurs millions de pixels.

Le dispositif peut comporter au moins une source d'éclairage agencée pour illuminer une partie à imager d'une portion périphérique de l'objet, en particulier par réflexion d'un faisceau lumineux émis par la source d'éclairage selon un axe d'éclairement peu incliné par rapport à l'axe de la première optique réflective, par la première optique réflective.

Le dispositif peut comporter par ailleurs au moins une source de pointage lumineux agencée pour former un repère lumineux sur une partie à imager d'une portion périphérique de l'objet, en particulier par réflexion d'un second faisceau lumineux émis par la source de pointage selon un axe de pointage peu incliné par rapport à l'axe de la première optique réflective, par la première optique réflective.

En particulier, le second faisceau lumineux de pointage peut être un faisceau plat apte à former un repère lumineux en forme de segment de droite.

Ce faisceau réfléchi par la première optique réflective peut s'étendre dans un plan dont la normale est légèrement inclinée par rapport à l'axe optique, selon un angle d'inclinaison déterminé, afin de permettre des opérations de triangulation. Une autre configuration peut être choisie pour éviter des recouvrement potentiels de faisceaux ; elle consiste à générer un faisceau dans un plan incluant sensiblement l'axe optique et permettant des mesures de distance le long de l'axe de l'objet et donc son inclinaison.

La trace du repère lumineux de pointage dans l'image obtenue de l'objet peut être utilisée pour déterminer les distances séparant respectivement la première optique réflective (et/ou la fenêtre transparente) des points de la surface périphérique de l'objet.

L'association de cette information de distance aux points d'une image obtenue peut ainsi permettre d'obtenir une « pseudo image » (c.à.d. un modèle) à trois dimensions de la surface périphérique de l'objet.

Le dispositif peut également traiter des recouvrements de deux images réfléchies par une surface réfléchissante déterminée de l'optique réflective, et respectivement captées par deux capteurs voisins/adjacents, et permettre ainsi la construction d'un modèle 3D de la surface périphérique de l'objet, par effet stéréoscopique.

S'il est possible de combiner les informations de distance entre les mesures obtenues par la source de pointage et les mesures stéréoscopiques, il est également possible d'utiliser des sources de pointage générant un ou plusieurs points lumineux répartis aléatoirement pour créer sur l'objet un motif lumineux unique facilement détectable par deux capteurs voisins et simplifiant ainsi les mesures stéréoscopiques.

Le dispositif peut comporter au moins une unité de traitement de signaux et/ou données qui est reliée au(x) capteur(s) d'images et est agencée (en particulier programmée) pour procéder à une partie au moins des traitements d'images requis pour déterminer une propriété de l'objet à partir des images acquises par le(s) capteur(s) ou imageur(s).

Le dispositif peut comporter en outre une unité de stockage de données reliée à l'unité de traitement de données et agencée pour enregistrer des données d'images délivrées par cette unité de traitement.

Le dispositif peut également comporter une batterie - ou autre source d'énergie électrique - agencée pour alimenter le(s) capteur(s) d'images, la (ou les) source(s) d'éclairage ou de pointage, l'unité de traitement de données, et l'unité de stockage de données.

L'invention peut notamment être appliquée à la prise d'images de parties de végétaux, sur leur lieu de culture, sans nécessiter une séparation de la partie à imager, du reste du végétal.

L'invention permet d'obtenir un dispositif d'imagerie compact et léger, qui peut être autonome, et qui peut être facilement porté et déplacé par un être humain, par un véhicule ou robot porteur, ou par tout autre dispositif, pour être engagé autour de l'objet à imager.

D'autres aspects, caractéristiques, et avantages de l'invention apparaissent dans la description suivante qui se réfère aux figures annexées et illustre, sans aucun caractère limitatif, des modes préférés de réalisation de l'invention.

### BREVE DESCRIPTION DES FIGURES

La figure 1 illustre schématiquement un dispositif de prise d'images, en vue en coupe longitudinale dans un plan contenant l'axe de symétrie de l'optique réflective du dispositif.
La figure 2 illustre schématiquement le dispositif de prise d'images illustré figure 1 et engagé autour d'une portion terminale d'un végétal telle qu'un épi de maïs.
La figure 3 illustre schématiquement le dispositif de prise d'images illustré figure 1 et engagé autour d'une portion intermédiaire (médiane) du végétal illustré figure 2.
La figure 4 illustre schématiquement un autre dispositif de prise d'images, en vue en coupe longitudinale dans un plan contenant l'axe de symétrie de l'optique réflective du dispositif, qui est engagé autour d'un objet de forme allongée qui s'étend au travers du dispositif de prise d'images.
La figure 5 illustre schématiquement une optique réflective et deux fenêtres tubulaires transparentes d'un dispositif de prise d'images, en vue en coupe transversale, perpendiculairement à l'axe de l'optique réflective et des fenêtres tubulaires.
La figure 6 illustre schématiquement une optique réflective entourant une fenêtre tubulaire transparente d'un dispositif de prise d'images, en vue en coupe transversale.
La figure 7 illustre schématiquement, en vue transversale également, une optique réflective entourant une fenêtre tubulaire transparente, ainsi que les capteurs d'images et les sources lumineuses d'éclairage et de pointage d'un dispositif de prise d'images.
La figure 8 illustre schématiquement, en vue en coupe longitudinale, une variante du dispositif de prise d'images illustré figure 1, qui comporte un dispositif de repliement du faisceau réfléchi par l'optique réflective entourant l'objet à imager.
La figure 9 illustre schématiquement des moyens de traitement d'images reliés à plusieurs capteurs d'images d'un dispositif de prise d'images.

### DESCRIPTION DETAILLEE DE L'INVENTION

Sauf indication explicite ou implicite contraire, des éléments ou organes - structurellement ou fonctionnellement - identiques ou similaires, sont désignés par des repères identiques sur les différentes figures.

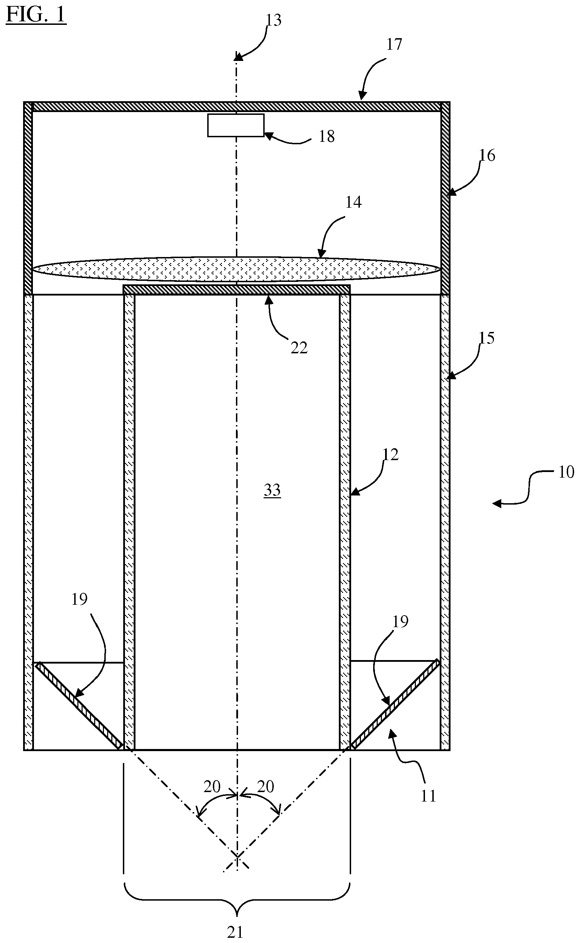

Par référence aux figures 1 à 3 notamment, l'appareil 10 de prise d'images comporte une optique 11 réflective qui est percée d'un évidement central 21 et entoure une fenêtre interne 12.

L'optique 11 est entourée par une fenêtre externe 15.

Les fenêtres 12, 15 peuvent être réalisées dans un matériau transparent tel que du verre. Alternativement, la fenêtre externe 15 peut être opaque.

Ces fenêtres présentent une forme tubulaire, en particulier une forme cylindrique de section circulaire, et dont l'axe 13 forme l'axe longitudinal et de symétrie générale du dispositif 10.

La fenêtre 12 s'étend en regard de l'optique 11 et présente une hauteur (mesurée selon l'axe 13) qui est au moins égale à celle de l'optique 11.

L'optique 11 est formée d'au moins un miroir - ou surface réfléchissante-19 qui est incliné(e) par rapport à l'axe 13 d'un angle aigu 20 qui est ici sensiblement égal à 45 degrés, de sorte que la surface 19 est dirigée vers l'axe 13 de l'appareil.

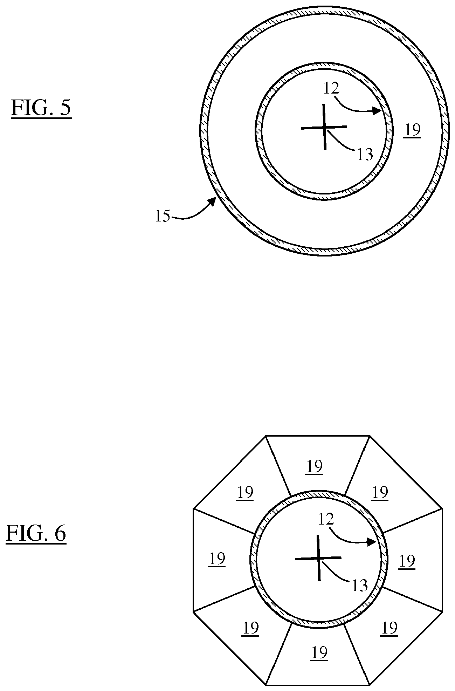

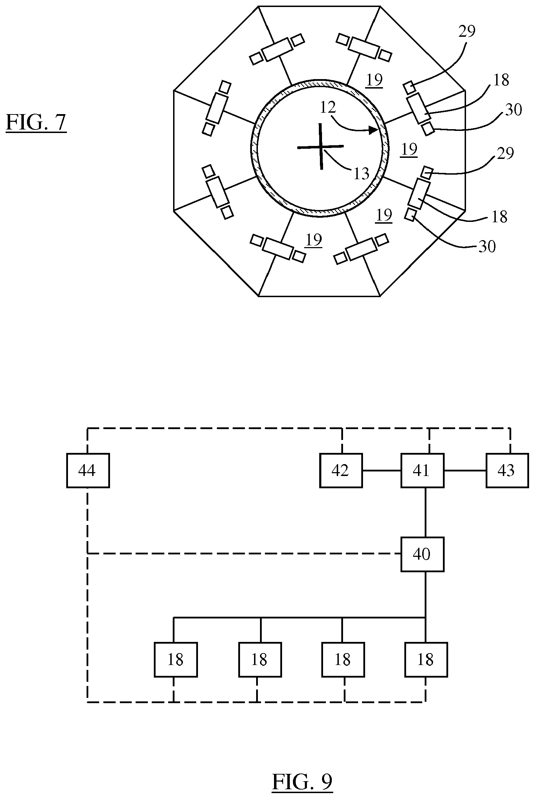

L'optique 11 peut comporter une surface 19 gauche unique, de forme tronconique comme illustré figure 5, ou plusieurs surfaces 19 planes de contour trapézoïdal curviligne, comme illustré figures 6 et 7.

L'appareil 10 comporte en outre un imageur comportant un capteur 18 d'images et un objectif 14 schématiquement représenté sous la forme d'une lentille biconvexe.

L'axe optique commun au capteur 18 et à l'objectif 14 est ici confondu avec l'axe 13 de l'appareil 10.

L'extrémité supérieure de la fenêtre tubulaire interne 12 est ici fermée par une paroi opaque 22 qui s'étend en regard de la partie centrale de l'objectif 14.

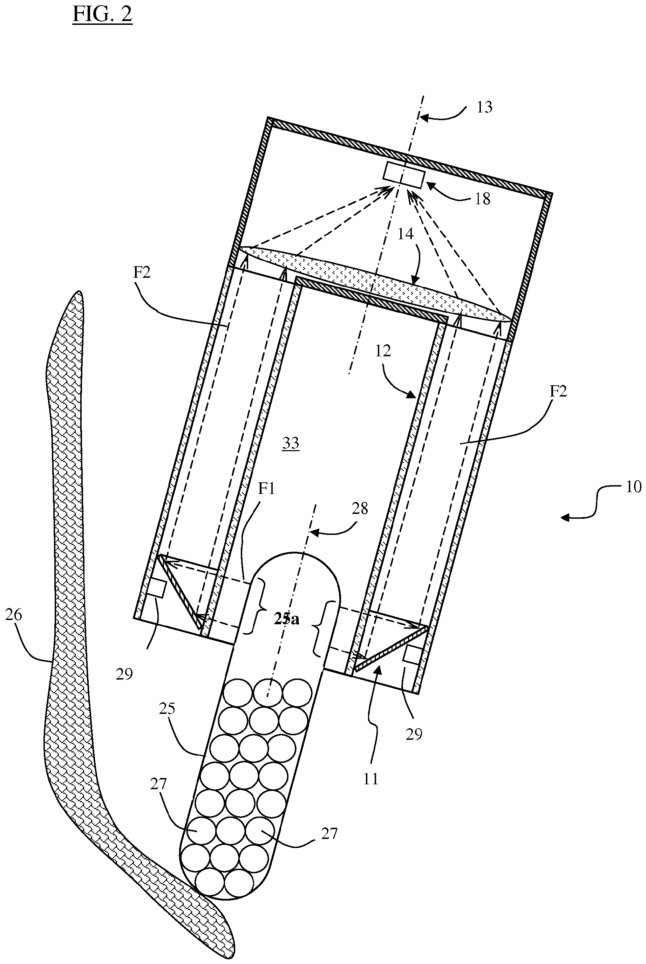

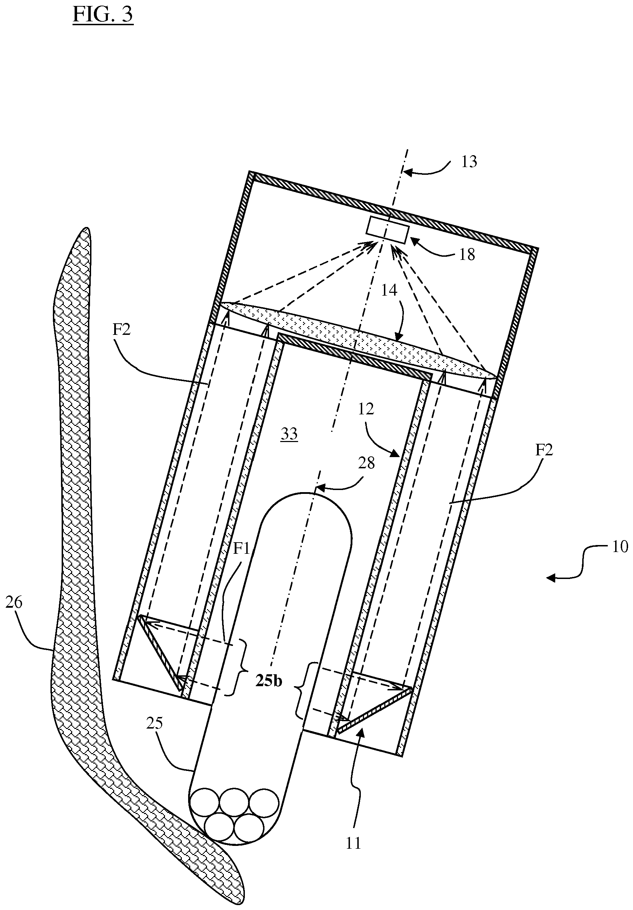

La fenêtre 12 et la paroi 22 délimitent ainsi une cavité 33 de forme allongée (cylindrique) selon l'axe 13, dont la partie inférieure est entourée par la fenêtre 12 et par l'optique 11, et qui peut recevoir un objet à imager comme illustré dans les figures 2 et 3.

Le dispositif 10 comporte en outre une paroi opaque 16 prolongeant la fenêtre 15 et fermée par une paroi 17 pour former un boîtier recevant le capteur 18 et l'objectif 14.

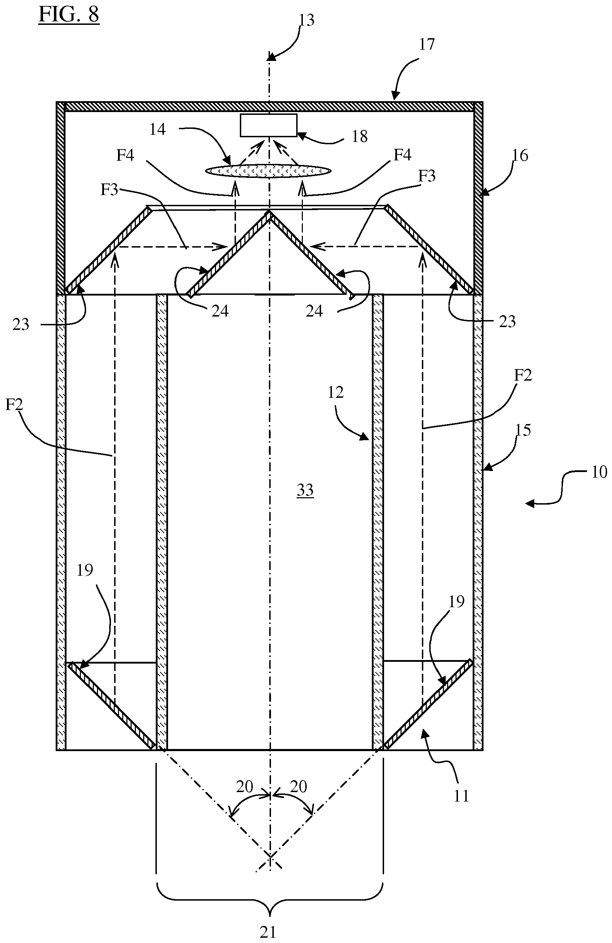

Dans la variante de réalisation illustrée figure 8, le dispositif 10 comporte un dispositif de repliement de faisceau disposé sur le trajet optique entre l'imageur 14, 18 et la (ou les) surface(s) réfléchissante(s) 19 de l'optique 11.

Ce dispositif de repliement comporte une seconde optique réflective évidée s'étendant autour d'un axe confondu avec l'axe 13 de la première optique réflective évidée, ainsi qu'une troisième optique réflective coaxiale avec les première et seconde optiques réflectives.

La seconde optique réflective comporte des surfaces 23 réfléchissantes dirigées vers l'axe 13, qui sont de préférence inclinées par rapport à cet axe d'un angle de 45 degrés environ.

En particulier, cette seconde optique peut être sensiblement symétrique de la première optique 11 par rapport à un plan transversal médian (qui est perpendiculaire à l'axe 13).

La seconde optique permet de réfléchir un faisceau F2 provenant des surfaces 19 et se propageant parallèlement à l'axe 13, pour produire un faisceau F3 se propageant radialement (par référence à l'axe 13), en direction de l'axe 13.

Les seconde et troisième optiques réflectives peuvent comporter des surfaces réfléchissantes respectives 23, 24 de forme tronconiques d'axe 13.

La (les) surface(s) 24 réfléchissante(s) de la troisième optique, qui s'étend(ent) en regard de la (des) surface(s) 23, permet(tent) de réfléchir le faisceau F3 pour produire un faisceau F4 se propageant parallèlement au faisceau F2, à plus faible distance de l'axe 13 et en direction de l'objectif 14 qui focalise ce faisceau F4 sur le capteur 18.

Sur les figures 2 et 3, le dispositif 10 est engagé autour d'une portion d'un épi 25 de maïs solidaire de la tige 26 d'un plant de maïs, pour permettre de déterminer une caractéristique de l'épi sans l'arracher du plant, par exemple pour compter les grains 27 (partiellement représentés) de l'épi à partir d'images de la périphérie de l'épi qui sont prises par le dispositif 10.

A cet effet, le dispositif 10 est disposé pour « coiffer » et entourer la partie supérieure de l'épi 25, ce dernier pénétrant en partie dans la cavité 33 par l'orifice de l'extrémité inférieure de la fenêtre tubulaire 12.

Le positionnement du dispositif 10 autour de l'épi 25 est de préférence effectué de façon à ce que la partie - ou « tranche » - supérieure 25a de l'épi, qui s'étend selon l'axe longitudinal 28 de l'épi, s'étendent sensiblement selon l'axe 13 de symétrie de l'optique 11 et de la cavité 33, de sorte que les différentes portions de la surface périphérique de la partie 25a de l'épi soient situées à sensiblement la même distance de la fenêtre 12, et par conséquent de l'optique 11.

Dans cette position relative de l'épi et du dispositif de prise d'images, le faisceau F1 de lumière réfléchie par la surface périphérique supérieure 25a de l'épi et se propageant sensiblement radialement, par référence aux axes 13 et 28, traverse la fenêtre 12 et est réfléchi par l'optique 11 pour former un faisceau F2 se propageant sensiblement parallèlement à l'axe 13.

Le faisceau F2 est focalisé par l'objectif 14 sur la surface sensible du capteur 18 pour former une image de la surface 25a qui est captée par le capteur 18 et peut être enregistrée et traitée par une unité de traitement (telle qu'un microprocesseur) intégrée au dispositif 10.

A cet effet, l'éclairement de la surface 25a peut résulter de la lumière « naturelle « (ambiante) traversant les fenêtres interne 12 et externe 15 du dispositif, et/ou de la lumière produite par une source d'éclairement intégrée à l'appareil 10.

Cette source d'éclairement peut par exemple être constituée par des diodes (LEDs) 29 régulièrement disposées autour de l'optique 11 (qui est alors semi transparente) et produisant des faisceaux lumineux traversant cette optique.

Après qu'une image de la surface périphérique (« tranche ») supérieure 25a ait été prise par un (ou des) capteur(s) 18, le dispositif peut être déplacé le long de l'axe 28 de l'épi, en direction de la base de l'épi, pour pouvoir prendre une image de la surface périphérique intermédiaire 25b de l'épi, comme dans la configuration illustrée figure 3.

Le dispositif 10 est de préférence maintenu sensiblement centré sur l(objet à imager, c.à.d. en maintenant sensiblement confondus les axes respectifs 13, 28 de l'optique 11 (et de la cavité 33) et de l'objet 25.

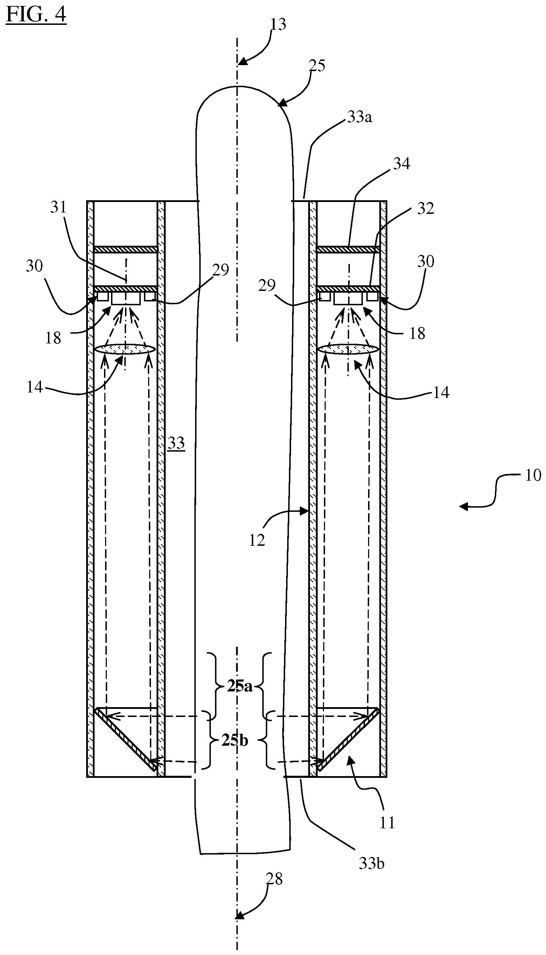

De préférence, le déplacement du dispositif et les prises de vue successives des portions 25a, 25b de l'objet qui sont espacées le long de l'axe 28, sont effectués de manière à ce que les images successivement prises se recouvrent deux à deux, comme illustré schématiquement figure 4, puis ces images sont traitées pour être raccordées en « empilant » leur recouvrement mutuel.

La fréquence d'acquisition de ces images peut être adaptée à la vitesse de déplacement du dispositif 10 le long de l'objet, pour obtenir ce recouvrement entre les images successives.

A cet effet, le dispositif peut comporter un capteur de mouvement, tel qu'au moins un accéléromètre ou autre capteur inertiel, qui est solidaire de la première optique réflective et/ou du corps du dispositif, de façon à être sensible à un déplacement du dispositif par rapport à un repère fixe - et donc par rapport à l'objet 25 -, et qui est relié aux moyens de traitement des images délivrées par le(s) capteur(s) 18, pour délivrer des signaux de mouvement permettant d'asservir l'acquisition d'images.

Dans le mode de réalisation illustré figure 4, le dispositif 10 comporte plusieurs imageurs comportant chacun un objectif 14 et un capteur 18 d'images. Les axes optiques 31 respectifs des imageurs sont distants de - et parallèles à - l'axe 13 de la première optique réflective, et disposés régulièrement autour de l'axe 13.

Les imageurs sont montés sur un support 32 annulaire (en forme de couronne) présentant un évidemment centré sur l'axe 13, et s'étendant dans un plan transversal, entre les fenêtres interne 12 et externe 15.

Les imageurs délivrent des signaux ou données aux moyens de traitement d'images qui sont montés sur un support 34 - tel qu'un circuit imprimé - également en forme de couronne s'étendant entre les fenêtres 12, 15.

Les objectifs 14 sont également fixés à un support (non représenté) annulaire s'étendant entre les fenêtres 12, 15.

Les supports annulaires 32, 34 contribuent à délimiter, avec la première optique réflective évidée, et le cas échéant avec la fenêtre transparente, une cavité 33 ouverte à ses deux extrémités 33a, 33b.

La cavité 33 est apte à recevoir l'objet qui traverse dans ce cas le dispositif de part en part, ce qui permet au dispositif d'être déplacé le long de l'objet à imager, sur une distance supérieure à la longueur du dispositif (et de la cavité).

Comme illustré figures 4 et 6, une source 29 d'éclairage est associée à chaque capteur 18 pour illuminer une partie à imager d'une portion périphérique de l'objet, par réflexion d'un faisceau lumineux émis par la source d'éclairage selon un axe d'éclairement qui peut être parallèle à l'axe 13, par la première optique réflective.

De la même façon, une source 30 de pointage lumineux est associée à chaque capteur 18 pour former un repère lumineux sur une partie à imager d'une portion périphérique de l'objet, par réflexion d'un faisceau lumineux émis par la source de pointage selon un axe de pointage qui peut être parallèle à l'axe 13 de l'optique 11, par l'optique 11.

Comme illustré figure 7, chaque capteur 18 dont l'axe optique croise une ligne de séparation de deux surfaces 19 adjacentes, peut ainsi recevoir une image d'une partie périphérique d'un objet, qui comporte deux parties d'images respectivement réfléchies par les deux surfaces 19 adjacentes.

Par ailleurs, deux capteurs 18 adjacents dont les axes optiques respectifs croisent respectivement deux bords opposés d'une surface 19 qui s'étend entre ces axes, permettent à ces deux capteurs d'obtenir, par vision stéréoscopique, une image à trois dimensions d'une partie périphérique d'un objet.

Par référence à la figure 9, le dispositif 10 comporte un premier circuit électronique 40 qui est relié à 4 capteurs 18 pour recevoir les données d'images captées par ces capteurs, et peut « fusionner » ces données et les délivrer à un second circuit électronique 41 procédant à des traitements d'image, notamment en fonction de signaux délivrés par un accéléromètre 43 relié au circuit 41, et enregistrant les images traitées dans une mémoire 42.

Une batterie 44 est reliée aux capteurs 18, 43, et aux circuits 40 à 42, pour les alimenter.

Le dispositif comporte de préférence en outre des moyens d'identification de l'objet et/ou des moyens d'association d'au moins une donnée d'identification à l'image (ou les images) d'un objet imagé par le dispositif.

L'identification de l'objet peut être « indirecte », pouvant par exemple consister en l'utilisation de données de géo localisation d'un objet pour identifier cet objet.

Dans ce cas, le dispositif peut comporter un récepteur de signaux de géo localisation émis par des satellites, tel qu'un récepteur GPS, qui est relié à l'unité 40, 41 de traitement de données, et les données de géo localisation du dispositif 10 - et par conséquent d'un objet entouré par le dispositif - peuvent être associées par l'unité 40, 41 - et/ou enregistrées avec l'image (ou les images) de cet objet prise(s) par le dispositif.

L'identification de l'objet peut aussi consister à associer des informations visuelles et/ou sonores à l'image prise d'un objet.

Pour associer des informations sonores à une image, le dispositif peut comporter un microphone relié à l'unité 40, 41 de traitement de données, cette unité assurant l'association de données sonores délivrées par le microphone à l'image (aux images) d'un objet déterminé, et/ou l'enregistrement de ces données sonores.

Pour associer des informations visuelles à une image, le dispositif peut comporter un capteur d'informations visuelles, par exemple un lecteur de codes barre, qui est relié à l'unité 40, 41 de traitement de données, cette unité assurant l'association de données visuelles délivrées par le capteur d'informations visuelles à l'image (aux images) d'un objet déterminé, et/ou l'enregistrement de ces données visuelles.

Le capteur d'informations visuelles peut être constitué par au moins un des capteurs 18 d'image, et l'unité 40, 41 de traitement peut alors être agencée pour extraire des informations visuelles des images prises, par exemple pour extraire des données d'identification de l'objet des images, en particulier pour extraire des données d'identification (par exemple code barre) portées par une étiquette apposée sur l'objet.

## Revendications

1. Dispositif (10) de prise d'images d'un objet (25), **caractérisé en ce qu'**il comporte :
- une optique réflective (11, 19) s'étendant autour d'un axe (13) de symétrie, présentant un évidemment (21) centré sur l'axe de symétrie, et comportant au moins une surface (19) réfléchissante inclinée par rapport à l'axe de symétrie (13), avec un angle (20) aigu d'inclinaison, de sorte que la surface (19) est dirigée vers l'axe de symétrie et peut entourer une partie (25a, 25b) au moins de l'objet (25); et
- au moins un imageur (14, 18) agencé pour capter au moins une image d'au moins une partie de l'objet qui est réfléchie par ladite au moins une surface réfléchissante (19) sensiblement parallèlement à l'axe de symétrie, lorsque l'optique réflective est disposée autour de ladite partie de l'objet.

2. Dispositif selon la revendication 1 qui comporte une fenêtre (12) transparente de forme cylindrique, en particulier de section circulaire, autour de laquelle s'étend l'optique réflective (11, 19).

3. Dispositif selon la revendication 1 ou 2 dans lequel l'optique réflective s'inscrit à l'intérieur d'un premier cylindre, l'évidemment s'inscrit à l'intérieur d'un second cylindre coaxial au premier cylindre, et le rayon du premier cylindre est inférieur ou égal au triple du rayon du second cylindre.

4. Dispositif selon l'une quelconque des revendications 1 à 3 dans lequel l'optique réflective comporte une unique surface réfléchissante (19) qui est gauche, en particulier de forme conique, et présente une symétrie de révolution selon un axe de révolution (13) confondu avec l'axe de symétrie.

5. Dispositif selon l'une quelconque des revendications 1 à 3 dans lequel l'optique réflective comporte plusieurs surfaces réfléchissantes (19), en particulier au moins trois ou quatre surfaces réfléchissantes planes de dimensions et de formes identiques, qui sont régulièrement disposées autour de l'axe de symétrie (13).

6. Dispositif selon l'une quelconque des revendications 1 à 5 dans lequel la (ou les) surface(s) réfléchissante(s) est (sont) inclinée(s) par rapport à l'axe de symétrie (ou de révolution) avec un angle d'inclinaison sensiblement égal à 45 degrés.

7. Dispositif selon l'une quelconque des revendications 1 à 6 dans lequel la (ou les) surface(s) réfléchissante(s) est (sont) partiellement réfléchissante(s) et partiellement transmissive(s) ou transparente(s).

8. Dispositif selon l'une quelconque des revendications 1 à 7 qui comporte un imageur unique comportant un objectif (14) dont le diamètre est au moins égal à la plus grande dimension extérieure de l'optique réflective (11), et dont l'axe optique (13) est voisin de et sensiblement parallèle à - en particulier sensiblement confondu avec - l'axe de symétrie, et qui comporte un dispositif de repliement de faisceau (F2 à F4) disposé sur le trajet optique entre l'imageur (14, 18) et la (ou les) surface(s) réfléchissante(s) (19).

9. Dispositif selon l'une quelconque des revendications 1 à 7 qui comporte plusieurs imageurs (14, 18) dont les axes optiques (31) respectifs sont distants de - et parallèles à - l'axe (13) de la première optique réflective, et de préférence disposés régulièrement autour de l'axe de la première optique réflective.

10. Dispositif selon la revendication 9 dans lequel les imageurs sont montés sur au moins un support (32) annulaire présentant un évidemment centré sur l'axe de la première optique réflective, ce support évidé contribuant à délimiter, avec la première optique réflective (11) évidée, et le cas échéant avec la fenêtre (12) transparente, une cavité (33) apte à recevoir l'objet.

11. Dispositif selon l'une quelconque des revendications 1 à 10 qui comporte au moins une source (29) d'éclairage agencée pour illuminer une partie à imager d'une portion périphérique (25a, 25b) de l'objet, par réflexion d'un faisceau lumineux émis par la source d'éclairage selon un axe d'éclairement peu incliné par rapport à l'axe (13) de la première optique réflective, par la première optique réflective (11).

12. Dispositif selon l'une quelconque des revendications 1 à 11 qui comporte au moins une source (30) de pointage lumineux agencée pour former un repère lumineux sur une partie à imager d'une portion périphérique (25a, 25b) de l'objet, par réflexion d'un second faisceau lumineux émis par la source de pointage selon un axe de pointage peu incliné par rapport à l'axe (13) de la première optique réflective, par la première optique réflective, le second faisceau lumineux de pointage pouvant être un faisceau plat apte à former un repère lumineux en forme de segment de droite.

13. Dispositif selon l'une quelconque des revendications 1 à 12 qui comporte au moins une unité (40, 41) de traitement de signaux et/ou données qui est reliée au(x) capteur(s) (18) d'images et est agencée (en particulier programmée) pour procéder à une partie au moins des traitements d'images requis pour déterminer une propriété de l'objet à partir des images acquises par le(s) capteur(s), et/ou agencée pour extraire des informations visuelles des images prises, en particulier pour extraire des données d'identification de l'objet des images, par exemple pour extraire des données d'identification portées par une étiquette apposée sur l'objet, et qui comporte une unité (42) de stockage de données reliée à l'unité (40, 41) de traitement de données et agencée pour enregistrer des données d'images délivrées par cette unité de traitement.

14. Dispositif selon l'une quelconque des revendications 1 à 13 qui comporte une batterie (44) agencée pour alimenter le(s) capteur(s) d'images (18), et le cas échéant la (ou les) source(s) (29, 30) d'éclairage ou de pointage, l'unité (40, 41) de traitement de données, et l'unité (42) de stockage de données.

15. Dispositif selon l'une quelconque des revendications 1 à 14 qui comporte des moyens de centrage de l'objet dans l'évidemment (21) de l'optique réflective, en particulier des organes d'appui sur l'objet qui sont montés mobiles par rapport à l'optique réflective selon des axes radiaux s'étendant dans un plan perpendiculaire à l'axe de l'optique réflective.

16. Procédé de prise d'images d'un objet (25) in situ, **caractérisé en ce que** :
- on entoure une partie (25a, 25b) au moins de l'objet qui s'étend selon un axe longitudinal (28), d'au moins une surface réfléchissante (19) d'un dispositif selon l'une quelconque des revendications 1 à 15, qui est inclinée par rapport à l'axe longitudinal (28), avec un angle (20) aigu d'inclinaison, de sorte que la surface (19) est dirigée vers l'axe longitudinal ; et
- on capte au moins une image d'au moins une partie périphérique de l'objet qui est réfléchie par ladite au moins une surface réfléchissante sensiblement parallèlement à l'axe longitudinal.

17. Procédé selon la revendication 16 dans lequel la prise d'image(s) est répétée plusieurs fois pour balayer la périphérie de l'objet, en déplaçant l'objet au travers d'un évidemment (21) d'une optique réflective (11) comportant la (ou les) surface(s) réfléchissante(s), entre deux prises d'image(s) successives, puis en raccordant deux à deux les images successivement prises.

18. Procédé selon la revendication 16 ou 17 dans lequel l'objet est une partie d'un végétal sur pied.

19. Procédé selon l'une quelconque des revendications 16 à 18 dans lequel, le dispositif (10) comportant deux capteurs (18) d'image adjacents dont les axes optiques (31) respectifs croisent respectivement deux bords opposés d'une surface (19) réfléchissante qui s'étend entre ces axes, on détermine, par vision stéréoscopique, une image à trois dimensions d'une partie périphérique de l'objet.

## Patentansprüche

1. Vorrichtung (10) zur Aufnahme von Bildern eines Objektes (25), **dadurch gekennzeichnet, dass** sie umfasst:
- eine Reflexionsoptik (11, 19), die um eine Symmetrieachse (13) angeordnet ist und eine um die Symmetrieachse zentrierte Durchbrechung (21) aufweist, und die mindestens eine reflektierende Fläche (19) aufweist, die in einem spitzen Neigungswinkel (20) relativ zur Symmetrieachse (13) geneigt ist, derart, dass die Fläche (19) der Symmetrieachse zugewandt ist und mindestens einen Teil (25a, 25b) des Objektes (25) umgeben kann, und
- mindestens ein Aufnahmegerät (14, 18), dafür eingerichtet, mindestens ein Bild mindestens eines Teils des Objektes aufzunehmen, der von der genannten mindestens einen reflektierenden Fläche (19) im Wesentlichen parallel zur Symmetrieachse reflektiert wird, wenn die Reflexionsoptik um den genannten Teil des Objektes herum angeordnet ist.

2. Vorrichtung nach Patentanspruch 1, die ein transparentes, zylindrisches Fenster (12) insbesondere kreisförmigen Querschnitts aufweist, um das herum die Reflexionsoptik (11, 19) angeordnet ist.

3. Vorrichtung nach Patentanspruch 1 oder 2, in der die Reflexionsoptik im Inneren eines ersten Zylinders liegt, die Durchbrechung im Inneren eines zum ersten Zylinder koaxialen zweiten Zylinders liegt, und der Radius des ersten Zylinders kleiner gleich dem Dreifachen des Radius des zweiten Zylinders ist.

4. Vorrichtung nach irgendeinem der Patentansprüche 1 bis 3, in der die Reflexionsoptik eine einzige reflektierende Fläche (19) umfasst, die windschief ist, insbesondere konisch, und Rotationssymmetrie um eine Drehachse (13), die mit der Symmetrieachse zusammenfällt, aufweist.

5. Vorrichtung nach irgendeinem der Patentansprüche 1 bis 3, in der die Reflexionsoptik mehrere reflektierende Flächen (19) umfasst, insbesondere mindestens drei oder vier ebene reflektierende Flächen identischer Abmessungen und Form, die gleichmäßig um die Symmetrieachse (13) angeordnet sind.

6. Vorrichtung nach irgendeinem der Patentansprüche 1 bis 5, in der die reflektierende(n) Fläche(n) relativ zur Symmetrie-(oder Dreh-) achse in einem Winkel geneigt ist/sind, der im Wesentlichen gleich 45° ist.

7. Vorrichtung nach irgendeinem der Patentansprüche 1 bis 6, in der die reflektierende(n) Fläche(n) teilreflektierend und teildurchlässig ist(sind).

8. Vorrichtung nach irgendeinem der Patentansprüche 1 bis 7, die ein einziges Aufnahmegerät aufweist, das ein Objektiv (14) aufweist, dessen Durchmesser mindestens gleich dem größten Außendurchmesser der reflektierenden Optik (11) ist und dessen optische Achse (13) der Symmetrieachse benachbart und im Wesentlichen parallel ist - insbesondere mit ihr im Wesentlichen zusammenfällt -, und das eine Strahlfaltungsvorrichtung (F2 bis F4) aufweist, die im Strahlengang zwischen dem Aufnahmegerät (14, 18) und der/den reflektierenden Fläche(n) (19) angeordnet ist.

9. Vorrichtung nach irgendeinem der Patentansprüche 1 bis 7, die mehrere Aufnahmegeräte (14, 18) aufweist, deren jeweilige optische Achsen (31) von der Achse (13) der ersten Reflexionsoptik fern - und zu ihr parallel - und vorzugsweise gleichmäßig um die Achse der ersten Reflexionsoptik angeordnet sind.

10. Vorrichtung nach Patentanspruch 9, in der die Aufnahmegeräte auf mindestens einem ringförmigen Träger (32) montiert sind, der eine um die Achse der ersten Reflexionsoptik (11) zentrierte Durchbrechung aufweist, wobei dieser durchbrochene Träger dazu beiträgt, mit der durchbrochenen ersten Reflexionsoptik (11) und gegebenenfalls mit dem transparenten Fenster (12) einen Hohlraum (33) zu umgrenzen, der geeignet ist, das Objekt aufzunehmen.

11. Vorrichtung nach irgendeinem der Patentansprüche 1 bis 10, die mindestens eine Beleuchtungslichtquelle (29) umfasst, dafür angeordnet, einen aufzunehmenden Teil eines Peripheriebereichs (25a, 25b) des Objektes durch Reflexion eines Lichtstrahles, der von der Beleuchtungslichtquelle längs einer wenig relativ zur Achse (13) der ersten Reflexionsoptik geneigten Beleuchtungsachse ausgestrahlt wird, durch die erste Reflexionsoptik (11) zu beleuchten.

12. Vorrichtung nach irgendeinem der Patentansprüche 1 bis 11, die mindestens eine Lichtzeigerquelle (30) aufweist, die dafür eingerichtet ist, durch Reflexion eines zweiten Lichtstrahles, der von der Lichtzeigerquelle längs einer relativ zur Achse (13) der ersten Reflexionsoptik wenig geneigten Achse durch die erste Reflexionsoptik ausgestrahlt wird, eine Lichtmarke auf einem aufzunehmenden Teil eines Peripheriebereiches (25a, 25b) des Objektes auszubilden, wobei der zweite Zeigerlichtstrahl ein flacher Strahl sein kann, der geeignet ist, eine Lichtmarkierung in Form eines Geradenabschnittes zu auszubilden.

13. Vorrichtung nach irgendeinem der Patentansprüche 1 bis 12, die mindestens eine Signal- und/oder Datenverarbeitungseinheit (40, 41) umfasst, die mit dem/n Bildaufnahmesensor(en) (18) verbunden ist und dafür eingerichtet (insbesondere programmiert) ist, mindestens einen Teil der Verarbeitung von Bildern vorzunehmen, der erforderlich ist, um aus den von dem/n Sensor(en) erfassten Bildern eine Eigenschaft des Objektes zu bestimmen, und/oder dafür eingerichtet, den aufgenommenen Bildern visuelle Informationen zu entnehmen, insbesondere zur Entnahme von Daten zur identifizierung des Objektes der Bilder, beispielsweise zur Entnahme der Identifizierungsdaten, die ein Etikett trägt, das auf dem Objekt angebracht ist, und die eine mit der Datenverarbeitungseinheit (40, 41) verbundene Datenspeichereinheit (42) umfasst und dafür eingerichtet ist, Bilddaten zu speichern, die von dieser Verarbeitungseinheit ausgegeben werden.

14. Vorrichtung nach irgendeinem der Patentansprüche 1 bis 13, die eine Batterie (44) umfasst, die dafür angeordnet ist, den/die Bildsensor(en) (18) und gegebenenfalls die Beleuchtungs- oder Zeigerlichtquellen (29, 30), die Datenverarbeitungseinheit (40, 41) und die Datenspeichereinheit (42) zu versorgen,

15. Vorrichtung nach irgendeinem der Patentansprüche 1 bis 14, die Mittel zum Zentrieren des Objektes in der Durchbrechung (21) der Reflexionsoptik aufweist, insbesondere Organe zum Drücken auf das Objekt, die relativ zur Reflexionsoptik längs radialer Achsen beweglich montiert sind, die in einer Ebene senkrecht zur Achse der Reflexionsoptik liegen.

16. Verfahren zur Aufnahme von Bilder von einem Objekt (25) in situ, **dadurch gekennzeichnet, dass**
- mindestens ein Teil (25a, 25b) des Objektes, der sich längs einer Längsachse (28) erstreckt, mit mindestens einer reflektierenden Fläche (19) einer Vorrichtung nach irgendeinem der Patentansprüche 1 bis 15, die relativ zur Längsachse (28) in einem spitzen Neigungswinkel (20) geneigt ist, derart, dass die Fläche (19) der Längsachse zugewandt ist, umgeben wird, und
- mindestens ein Bild mindestens eines Peripherieteils des Objektes, der von der genannten mindestens einen reflektierenden Fläche im Wesentlichen parallel zur Längsachse reflektiert wird, erfasst wird.

17. Verfahren nach Patentanspruch 16, in dem die Bildaufnahme mehrfach wiederholt wird, um die Peripherie des Objektes abzutasten, in dem das Objekt zwischen zwei aufeinanderfolgenden Bildaufnahmen durch eine Durchbrechung (21) einer Reflexionsoptik (11) versetzt wird, die die reflektierende(n) Fläche(n) umfasst, und dann die nacheinander aufgenommenen Bilder jeweils zusammengesetzt werden.

18. Verfahren nach Patentanspruch 16 oder 17, in dem das Objekt Teil einer Pflanze mit Wurzeln ist.

19. Verfahren nach irgendeinem der Patentansprüche 16 bis 18, in dem, wenn die Vorrichtung (10) zwei einander anschließende Bildsensoren (18) aufweist, deren jeweilige optische Achsen (31) zwei gegenüberliegende Ränder einer reflektierenden Fläche (19) durchqueren, die zwischen diesen Achsen liegt, stereoskopisch ein dreidimensionales Bild eines Peripherieteils des Objektes erstellt wird.

## Claims

1. A device (10) for taking images of an article (25), the device being **characterized in that** it comprises:
- a reflective optical system (11, 19) extending around an axis of symmetry (13), having a recess (21) centered on the axis of symmetry and including at least one reflecting surface (19) inclined relative to the axis of symmetry (13), at an acute angle (20) of inclination, whereby the surface (19) is facing towards the axis of symmetry and can surround at least a portion (25a, 25b) of the article (25); and
- at least one imager (14, 18) arranged to capture at least one image of at least a portion of the article as reflected by said at least one reflecting surface (19) substantially parallel to the axis of symmetry when the reflective optical system is arranged around said portion of the article.

2. A device according to claim 1, including a transparent window (12) of cylindrical shape, in particular of circular section, around which the reflective optical system (11, 19) extends.

3. A device according to claim 1 or claim 2, wherein the reflective optical system extends inside a first cylinder, the recess extending inside a second cylinder coaxial with the first cylinder, and the radius of the first cylinder is less than or equal to three times the radius of the second cylinder.

4. A device according to any one of claims 1 to 3, wherein the reflective optical system includes a single reflecting surface (19) that is warped, in particular of conical shape, and presents symmetry of revolution about an axis of revolution (13) coinciding with the axis of symmetry.

5. A device according to any one of claims 1 to 3, wherein the reflective optical system includes a plurality of reflecting surfaces (19), in particular at least three or four plane reflecting surfaces of identical shape and dimensions, which surfaces are regularly arranged around the axis of symmetry (13).

6. A device according to any one of claims 1 to 5, wherein the reflecting surface(s) is/are inclined relative to the axis of symmetry (or of revolution), at an angle of inclination that is substantially equal to 45 degrees.

7. A device according to any one of claims 1 to 6, wherein the reflecting surface(s) is/are partially reflecting and partially transmissive or transparent.

8. A device according to any one of claims 1 to 7, having a single imager with a lens (14) of diameter not less than the largest outside dimension of the reflective optical system (11) and of optical axis (13) close to and substantially parallel to - and in particular substantially coinciding with - the axis of symmetry, and including a beam-folder device (F2 to F4) placed on the light path between the imager (14, 18) and the reflecting surface (s) (19).

9. A device according to any one of claims 1 to 7, having a plurality of imagers (14, 18) with respective optical axes (31) that are spaced apart from - and parallel to - the axis (13) of the first reflective optical system, and that are preferably arranged regularly around the axis of the first reflective optical system.

10. A device according to claim 9, wherein the imagers are mounted on at least one annular support (32) presenting a recess centered on the axis of the first reflective optical system, the hollowed-out support cooperating with the first hollowed-out reflective optical system (11), and where appropriate with the transparent window (12), to contribute to defining a cavity (33) suitable for receiving the article.

11. A device according to any one of claims 1 to 10, including at least one light source (29) arranged to illuminate a portion (25a, 25b) for imaging of a periphery of the article, by using the first reflective optical system (11) to reflect a light beam emitted by the light source along a lighting axis that is inclined little, if at all, relative to the axis (13) of the first reflective optical system.

12. A device according to any one of claims 1 to 11, including at least one aiming light source (30) arranged to form a light mark on a portion (25a, 25b) for imaging of the periphery of the article, by using the first reflective optical system to reflect a second light beam emitted by the aiming source along an aiming axis that slopes little, if at all, relative to the axis (13) of the first reflective optical system, the second light beam for aiming possibly being a flat beam suitable for forming a light mark in the form of a line segment.

13. A device according to any one of claims 1 to 12, including at least one signal and/or data processor unit (40, 41) that is connected to the image sensor(s) (18) and arranged (in particular programmed) to perform at least some of the image processing required for determining a property of the article from the images acquired by the sensor(s), and/or arranged to extract visual information from the images taken, in particular in order to extract data for identifying the article in the images, e.g. in order to extract identification data carried by a label placed on the article, and including a data storage unit (42) connected to the data processor unit (40, 41) and arranged to store image data delivered by the processor unit.

14. A device according to any one of claims 1 to 13, including a battery (44) arranged to power the image sensor(s) (18), and the illumination or aiming light source(s) (29, 30), if any, the data processor unit (40, 41), and the data storage unit (42).

15. A device according to any one of claims 1 to 14, including means for centering the article in the recess (21) in the reflective optical system, in particular members for pressing against the article and that are mounted to move relative to the reflective optical system along radial axes extending in a plane perpendicular to the axis of the reflective optical system.

16. A method of taking images in situ of an article (25), the method being **characterized by** the following steps:
- surrounding at least a portion (25a, 25b) of the article that extends along a longitudinal axis (28), by at least one reflecting surface (19) of a device according to any one of claims 1 to 15, the surface (19) being inclined relative to the longitudinal axis (28), at an acute angle (20) of inclination, whereby the surface (19) is directed towards the longitudinal axis; and
- capturing at least one image of at least a peripheral portion of the article, which image is reflected by said at least one reflecting surface substantially parallel to the longitudinal axis.

17. A method according to claim 16, wherein image taking is repeated several times in order to scan the periphery of the article by moving the article through a recess (21) in a reflective optical system (11) including the reflecting surface(s) between two successive image takes, and then splicing together in pairs the images as taken in succession.

18. A method according to claim 16 or claim 17, wherein the article is a portion of a growing plant.

19. A method according to any one of claims 16 to 18, wherein the device (10) has two adjacent image sensors (18) with respective optical axes (31) respectively crossing two opposite edges of a reflecting surface (19) that extends between said axes, and a three-dimensional image of a peripheral portion of the article is determined by stereoscopic vision.
